Europäisches Patentamt

(19) European Patent Office

Office européen des brevets

(11) Publication number: **0 043 169**
**B1**

(12) **EUROPEAN PATENT SPECIFICATION**

(45) Date of publication of patent specification: **12.10.83**

(21) Application number: **81200723.5**

(22) Date of filing: **26.06.81**

(51) Int. Cl.³: **C 12 N 9/24**, C 12 P 19/14, C 12 N 11/08, C 12 N 11/04 //C12R1/66

(54) Inulinase.

(30) Priority: **27.06.80 NL 8003723**

(43) Date of publication of application:
**06.01.82 Bulletin 82/1**

(45) Publication of the grant of the patent:
**12.10.83 Bulletin 83/41**

(84) Designated Contracting States:
**BE DE FR GB NL**

(56) References cited:
GB - A - 1 444 539
NL - A - 7 906 064
US - A - 4 110 163

CHEMICAL ABSTRACTS, vol. 30, November 20, 1936 column 8254,7 Columbus, Ohio, USA, NUGGEHALLI K. et al. "Inulinase"

CHEMICAL ABSTRACTS, vol. 86, no. 17, April 25, 1977, page 188, abstract 116810u Columbus, Ohio, USA, KIERSTAN, M. et al. "The immobilization of microbial cells, subcellular organelles, and enzymes in calcium alginate gels"

(73) Proprietor: STAMICARBON B.V.
Postbus 10
NL-6160 MC Geleen (NL)

(72) Inventor: Peters, Peter Josephus Hubertus
Kochstraat 6
NL-6164 HB Geleen (NL)
Inventor: Kerkhoffs, Pieter Laurens
van Goghstraat 1
NL-6165 VG Geleen (NL)

(74) Representative: Van de Panne, Vitus Nicolaas et al, Octrooibureau DSM Postbus 9
NL-6160 MA Geleen (NL)

(56) References cited:
CHEMICAL ABSTRACTS, vol. 90, no. 13, March 26, 1979, page 183, abstract 99073u Columbus, Ohio, USA, NAKAMURA, T. et al. "Studies on microbial inulase. Part V. General properties of an extracellular inulase (P—II) from Aspergillus sp."

CHEMICAL ABSTRACTS, vol. 88, no. 19, May 8, 1978, page 177, abstract 132445a Columbus, Ohio, USA, NAKAMURA, T. et al. "Studies on microbial inulase Part II General properties of extracellular inulase from Penicillium"

Courier Press, Leamington Spa, England.

(56) References cited:
CHEMICAL ABSTRACTS, vol. 82, no. 7,
February 17, 1975, page 317, abstract 41845r
Columbus, Ohio, USA, DOBROLINSKAYA, G. M.
et al. "Biosynthesis of beta-fructofuranosidase
by fungi of the genus Aspergillus"

## Inulinase

The invention relates to enzyme preparations with inulinase activities, their preparation and application.

It is known in the art that fructose polymers (inulins) can be hydrolyzed to fructose by means of enzymes. The enzyme can be obtained from cultures of, among others, *Saccharomyces fragilis, Candida kefyr, Aspergillus niger* and *Fusarium roseum.* In order to be able to carry out the hydrolysis on an industrial scale, an enzyme preparation with a maximum activity, a great stability and an optimum effect in a weakly acid medium is desirable. The purpose of the invention is such an enzyme preparation.

According to the invention an enzyme preparation with inulinase activity is obtained from a culture of *Aspergillus phoenicis.*

Thus an enzyme preparation with a high inulinase activity and a very low Michaelis constant, showing an optimum inulinase activity in a favourable pH range, can be obtained. Furthermore, the thermal stability is great, and the enzyme shows only little inhibition by heavy-metal ions and no substrate inhibition. A further advantage is that in cultures of *Aspergillus phoenicis* the enzyme with inulinase activity is present extracellularly.

Particularly suitable for the preparation of an enzyme preparation with inulinase activity is *Aspergillus phoenices* CSB 294.80, filed by applicant with the Centraal Bureau voor Schimmelculturen at Baarn. For a description of *Aspergillus phoenicis* reference is here made to 'The Genus Aspergillus' by K. B. Raper and D. I. Fennel (Baltimore 1965) pages 307—309.

The mould can be pre-cultured on a usual substrate. The spores are harvested and transferred into a stirred culture vessel suitable for aerobic cultures. A usual medium can be applied consisting of an aqueous buffered solution containing an assimilable nitrogen and carbon source and a phosphor source. Spore elements can be added, if so desired. A suitable medium consists of, for instance, a sterilized solution of 20 g yeast extract, 1 g sodium ammonium phosphate, 0.5 g ammonium sulphate and 15 g inulin in one litre of water. The addition of glucose is less desirable, because it is converted into acids. Owing to the presence of a small quantity of inulin in the medium, the formation of the desired enzyme is accelerated. The pH of the medium must be kept at a value of at least 4.0. At a lower pH hardly any or no inulinase is formed. The pH is preferably kept at a value of between 5 and 7. Particularly suitable is a pH of between 5.5 and 6.0. The temperature of the culture may be between 20°C and 40°C. Most preferably a temperature of between 25°C and 30°C is applied. Culturing is carried out under aerobic conditions. Of the total quantity of inulinase formed, about 85% is present extracellularly.

After a culture time of 4 to 10 days to liquid phase will contain a sufficient quantity of the desired enzyme. Thus after 8 days of culturing at 28°C and a pH of between 5.5 and 6.0 an activity of 36 I.U./ml culture medium was found. 1 I.U. is defined as the production of 1 micromole product per minute under de assay conditions. By removing the cells, a solution of the enzyme is obtained which can even be further concentrated, if so desired, by distilling off water at reduced pressure. The enzyme can be obtained in solid form by applying the usual methods, such as freeze drying, spray drying or drying of the solution by evaporation.

The enzyme can be classified as inulinase (or inulase) EC 3.2.1.7.

The free enzyme obtained according to the invention has a maximum activity at 60° or somewhat higher and at a pH of between 3.5 and 4.5. The Michaelis constant $K_m$ is about $2 \times 10^{-6}$ M/l, which is considerably lower than the value for other enzymes with inulinase activity. This means that the enzyme obtained according to the invention is better capable of fully hydrolyzing inulin than other enzymes known in the art. The enzyme, further-more, shows little sensitivity to inhibition by heavy metal ions, such as Hg(II). From literature it is known that the enzyme with inulinase activity obtained from cultures of *Aspergillus niger* is very sensitive to inhibition by Hg(II). The enzyme obtained according to the invention, furthermore, has a low invertase activity, unlike the enzymes obtained from the cultures of yeasts.

The enzyme can be used in the free, watersoluble form, but for industrial uses it is preferably brought into a water-insoluble, immobilized form. The immobilization can be effected by applying usual methods. The enzyme can be enclosed in a polymer matrix, for instance in a polyacrylamide gel, an alginate gel or in cellulose acetate. It can be adsorbed to an ion exchanger, although this form of immobilization is reversible and is, therefore, not preferred. It is, furthermore, possible to cross-link the enzyme, whether or not in the presence of a filler, with a bifunctional reagent, such as dialdehyde, a dicarboxylic acid or anhydride thereof or a diisocyanate. The enzyme can also be reacted with a polymer containing reactive groups such as, for instance, polyacrylicacid anhydride, a styrene-maleic anhydride copolymer or a poly-(-methylvinylether)-maleic anhydride copolymer. The enzyme can furthermore be reacted with a synthetic or natural solid provided with reactive groups. As solid, glass, steel, silica, alumina, an ion exchanger resin, pumice, sponge, activated carbon or mineral can be used, among other things. The above and other methods have been described in detail in literature.

It is favourable that the immobilized enzyme retains a low Michaelis constant and has an optimum activity over a wide pH range of 4.0 to 5.5 or somewhat higher. This means that fluctuations in the pH during the hydrolysis are permissible, which facilitates the application of the immobilized enzyme.

The enzyme obtained by applying the process according to the invention can be used in free form for the hydrolysis of inulin recovered from vegetable material. The extraction from the vegetable material can also be combined with the hydrolysis in the way described in the Dutch patent application 7811389 laid open for public inspection. For the hydrolysis of an inulin-containing solution preference, however, is given to the use of the enzyme in immobilized form. The immobilized enzyme can be used in a fixed bed or fluidized bed or in a stirred tank reactor.

The hydrolysis of inulin is preferably carried out at a pH of between 4.0 and 6.0, a temperature of between 20°C and 65°C and an inulin concentration of between 5 and 30% by weight in respect of the substrate solution. In this connection inulin is understood to mean all oligomers and polymers occuring in nature, consisting of one unit derived from glucose and 2 or more units derived from fructose. Inulin can be obtained from, among other things, dahlia tubers and the roots of jerusalem artichoke and chicory.

### Example I

The strain of *Aspergillus phoenicis* CBS 294.80 was inoculated on the following, pre-sterilized, fixed medium: 30 g malt extract, 5 g bactopepton (of Oxoid Ltd), 0.5 g ammonium sulphate, 5 g inulin, (product code 2327 of J. T. Baker Chemicals, Deventer, Holland) 0.2 g KC1, 20 g agar, 1 l water. After a culture time of 7 days at 28°C the spores formed were harvested.

A culture medium was prepared by sterilizing a solution of 20 g yeast extract, 1 g sodium ammonium phosphate, 0.5 g ammonium sulphate and 15 g inulin in 1 l water. The pH of the medium was 5.5. The spores thus obtained were inserted into the medium, upon which culturing was carried out for 7 days while stirring (200 rpm) at 28°C. The cells were subsequently removed by centrifugation. The remaining liquid contained the inulinase formed and had an activity of 36 I.U./ml. When the liquid was evaporated to dryness at 40°C and reduced pressure and the solid was subsequently dissolved again in water to the original volume, the activity of the liquid thus obtained was found to equal the original activity. Hence the thermal stability of the enzyme was good. The activity of the free enzyme depended on the pH, with an optimum at pH 3.5, and on the temperature, with an optimum at just over 60°C. The Michaelis constant $K_m$ of the free enzyme was 10 mg/l. As the molecular weight of the inulin used was about 5000, this corresponds with a $K_m$ of

about $2 \times 10^{-6}$ M/1. Addition of $HgCl_2$ to a quantity of 0.5% by weight (calculated in respect of the solution) had only little effect on the enzymatic activity.

### Example II

Into a column of a diameter of 15 cm and a length of 200 cm 25 l of an 0.5 M calcium chloride solution was fed which also contained 0.5% by weight of inulin and 0.13% by weight of a surface active agent (Tween-80). A homogeneous mixture was prepared of 750 ml cell-free culture medium obtained in the manner described in example I from a culture of *Aspergillus phoenicis* CBS 294.80, and 1750 ml 2% (wt) sodium-alginate solution. The mixture was slowly fed, in drops, into the top of the column via a precision metering device, in which process pellets of equal dimensions were formed, which sank to the bottom of the column. After the whole mixture had been fed, the pellets were filtered off and then washed, during stirring, with successively an 0.5 M calcium chloride solution, water and an 0.1 M acetate buffer (pH 5.0).

The immobilized enzyme preparation thus obtained was used for the hydrolysis of inulin. To this end 10 kg of a 10% (wt) inulin solution, which has been brought, with phosphate, to a pH of 5.0, was stirred for 1 hour at 50°C with the quantity of immobilized enzyme obtained above. By means of high pressure liquid chromatography it was shown that the inulin was fully hydrolyzed. The sugar solution formed was separated off by filtration. Of the sugars 97% by weight was found to consist of fructose.

### Example III

A phenolformaldehyde resin with hydroxyl groups (trade product Duolite S761 of Dia-Prosim, France) was thoroughly washed with, successively, ethanol, water, diluted lye, diluted hydrochloric acid, diluted lye and 0.15 M phosphate buffer. 25 ml of the resin was subsequently stirred for one hour at room temperature with a mixture of 25 ml 0.15 M phosphate buffer (pH 7.7) and 6 ml 25% by weight glutardialdehyde solution. The final pH was 6.5. The resin thus modified was filtered off and subsequently added to a mixture, cooled to 4°C, of 1.05 g enzyme solution, obtained in the manner described in example I, and 24 ml 0.15 M phosphate buffer. After stirring of the suspension for 20 hours at 4°C, the solid was filtered off and washed with a 1.5 M phosphate buffer (pH 6.5). By comparing the activity of the solution of the free enzyme with that of the immobilized enzyme preparation obtained in the manner described above it was found that the activity efficiency of the immobilization was 41.8%.

The immobilized enzyme preparation thus obtained was tested. It was found that the activity was of an optimum degree and virtually stable in the pH range between 4.5 and 6.0. The activity depended on the temperature, with an optimum at just over 60°C. The $K_m$ was 20 mg/l, corresponding with $4 \times 10^{-6}$ M/1 for the conversion of inulin with a molecular weight of about 5000.

Similar immobilized enzyme preparations could be obtained by starting from other resins such as, for instance, Duolite A—7 (a phenolformaldehyde resin containing amono groups).

## Claims

1. Enzyme preparation with inulinase activity, characterized in that it has been obtained from a culture of *Aspergillus phoenicis.*

2. Enzyme preparation according to claim 1, characterized in that it has been obtained from a culture of *Aspergillus phoenicis* CBS 294.80 or a mutant or variant thereof.

3. Enzyme preparation according to claims 1—2, characterized in that it has been obtained by culturing the mould in a, furthermore, usual aqueous medium at a pH ranging from 4.0 to 7.0 in the presence of inulin and absence of glucose and recovering the enzyme present in the culture medium.

4. Enzyme preparation according to claims 1—3, characterized in that the enzyme has been brought into a water-insoluble form by encapsulation in a calcium alginate gel.

5. Enzyme preparation according to claims 1—3, characterized in that the enzyme has been brought into a water-insoluble form by reacting the free enzyme with a resin obtained by reaction of an organic macroporous polymer containing amino and/or hydroxyl groups with glutardialdehyde.

6. Process for the hydrolysis of inulin in aqueous medium in the presence of an enzyme, characterized in that an enzyme preparation is used according to any one or more of the claims 1—5.

7. Process for the preparation of an enzyme preparation with inulinase activity by culturing a suitable micro-organism in a known manner and isolating the inulinase formed or making it otherwise accessible to a substrate, characterized in that a micro-organism belonging to the species *Aspergillus phoenicis* is used.

## Patentansprüche

1. Enzympräparat mit Inulinase-Aktivität, dadurch gekennzeichnet, dass es aus einer Kultur von *Aspergillus phoenicis* gewonnen wurde.

2. Enzympräparat nach Anspruch 1, dadurch gekennzeichnet, dass es aus einer Kultur von *Aspergillus phoenicis* CBS 294.80 oder einer Mutante oder Variante hiervon gewonnen wurde.

3. Enzympräparat nach Anspruch 1—2, dadurch gekennzeichnet, dass es gewonnen wurde durch Züchten des Schimmels in einem gebräuchlichen wässrigen Medium bei einem pH-Wert zwischen 4,0 und 7,0 in Anwesenheit von Inulin und in Abwesenheit von Glucose und durch anschliessende Gewinnung des im Zuchtmedium anwesenden Enzyms.

4. Enzympräparat nach Anspruch 1—3, dadurch gekennzeichnet, dass das Enzym durch Einkapselung in ein Calciumalginatgel in eine wasserunlösliche Form gebracht wurde.

5. Enzympräparat nach Anspruch 1—3, dadurch gekennzeichnet, dass das Enzym in eine, wasserunlösliche Form gebracht wurde, indem man das freie Enzym mit einem Harz reagieren liess, welches durch Reaktion eines organischen, makroporösen, Amino- und/oder Hydroxylgruppen enthaltenden Polymers mit Glutardialdehyd gewonnen wurde.

6. Verfahren zur Hydrolyse von Inulin in wässrigem Medium in Anwesenheit eines Enzyms, dadurch gekennzeichnet, dass ein Enzympräparat nach einem oder mehreren der Ansprüche 1—5 verwendet wird.

7. Verfahren zur Herstellung eines Enzympräparats mit Inulinase-Aktivität durch Züchtung eines geeigneten Mikroorganismus in gebräuchlicher Weise und durch Isolierung der gebildeten inulinase oder indem man dieses auf andere Weise für ein Substrat zugänglich macht, dadurch gekennzeichnet, dass ein Mikroorganismus verwendet wird, der zur Spezies *Aspergillus phoenicis* gehört.

## Revendications

1. Préparation d'enzyme avec activité d'inulinase, caractérisée en ce qu'elle est obtenue à partir d'une culture d'*Aspergillus phoenicis.*

2. Préparation d'enzyme selon la revendication 1, caractérisée en ce qu'elle est obtenue à partir d'une culture d'*Aspergillus phoenicis* CBS 294.80 ou d'un mutant ou d'une variation de celle-ci.

3. Préparation d'enzyme selon l'une des revendications 1 et 2, caractérisée en ce qu'elle est obtenue en cultivant la moisissure dans un milieu aqueux usuel à un pH situé entre 4,0 et 7,0, en présence d'inuline et en absence de glucose et en extrayant l'enzyme se trouvant dans le milieu de culture.

4. Préparation d'enzyme selon l'une des revendications 1 à 3, caractérisée en ce que l'enzyme est mise sous une forme insoluble dans l'eau, en l'encapsulant dans un gel d'alginate de calcium.

5. Préparation d'enzyme selon l'une des revendications 1 à 3, caractérisée en ce que l'enzyme est mise sous une forme non soluble dans l'eau, en faisant réagir l'enzyme libre sur une résine obtenue par la réaction d'un polymère organique macroporeux contenant des groupements amino et/ou hydroxyle avec du glutardialdéhyde.

6. Procédé d'hydrolyse d'inuline dans un milieu aqueux en présence d'une enzyme, caractérisé en ce qu'on utilise une préparation d'enzyme selon l'une ou plusieurs des revendications 1 à 5.

7. Procédé de fabrication d'une préparation d'enzyme avec activité d'inulinase en cultivant de façon connue un micro-organisme approprié et en isolant l'inulinase formée ou la rendant accessible d'une autre façon à un substrat, caractérisé en ce qu'on utilise un micro-organisme appartenant à l'espèce *Aspergillus phoenicis.*